# EUROPEAN PATENT APPLICATION

(11) **EP 3 485 804 A1**
(43) Date of publication of application: **22.05.2019**
(21) Application number: 18183027.4
(22) Date of filing: 11.07.2018
(51) Int. Cl.: A61B 5/00, A61B 5/024, A61B 5/0245, G06K 9/00

(54) **WEARABLE DEVICE CAPABLE OF RECOGNIZING DOZE-OFF STAGE AND RECOGNITION METHOD THEREOF**

(30) Priority: 20.11.2017 TW 106140054
(71) Applicant: Kinpo Electronics, Inc., New Taipei City 22201 (TW)
(72) Inventor: HARAIKAWA, Koichi, 22201 New Taipei City (TW); CHIEN, Jen-Chien, 22201 New Taipei City (TW); LIN, Yin-Tsong, 22201 New Taipei City (TW); HUNG, Tsui-Shan, 22201 New Taipei City (TW); HSIEH, Yi-Ta, 22201 New Taipei City (TW); LIN, Chien-Hung, 22201 New Taipei City (TW)
(74) Representative: Emde, Eric

(57) **Abstract**

A wearable device capable of recognizing doze-off stage including a processor and an electrocardiogram sensor is provided. The processor trains a neural network module. The processor is coupled to the electrocardiogram sensor. The electrocardiogram sensor is configured to generate an electrocardiogram signal. The processor performs a heart rate variability analysis operation and a R-wave amplitude analysis operation to analyze a heart beat interval variation of the electrocardiogram signal, so as to generate a plurality of characteristic values. The processor utilizes the trained neural network module to perform a doze-off stage recognition operation according to the characteristic values, so as to obtain a doze-off stage recognition result. In addition, a recognition method is also provided.

## Description

### Technical Field

The disclosure relates to a wearable device, and more particularly, to a wearable device capable of recognizing doze-off stage and a recognition method thereof.

### BACKGROUND

In the technical field for recognizing doze-off stage, the common sleep stage recognition for doze-off stage is usually conducted by utilizing a smart device with use of multiple physiological parameters such as brainwave, heartbeat, breathing or blood pressure so a doze-off stage recognition can be performed. Alternatively, a comparative recognition may be performed through an image processing to analyze variation of eye, head or mouth so a doze-off stage of a tester can be recognized. In other words, the common sleep stage recognition for the doze-off stage requires complicated accessories to be worn by the tester and requires analysis on a large amount of sense data. Consequently, the technique for recognizing the doze-off stage cannot be widely applied to various smart devices since the cost is overly high and the analysis procedure is complicated. In consideration of the above, providing a wearable device capable of effectively sensing a sleep stage of the tester in the doze-off stage having characteristics of convenience is one of important issues to be addressed in the field.

### SUMMARY

The disclosure provides a wearable device capable of recognizing doze-off stage and a method thereof, which can effectively recognize the doze-off stage of a wearer and provide characteristics of convenience.

A wearable device capable of recognizing doze-off stage of the disclosure includes a processor and an electrocardiogram sensor. The processor is configured to train a neural network module. The electrocardiogram sensor is coupled to the processor. The electrocardiogram sensor generates an electrocardiogram signal. The processor performs a heart rate variability analysis operation and a R-wave amplitude analysis operation to analyze a heart beat interval variation of the electrocardiogram signal, so as to generate a plurality of characteristic values. The processor utilizes the trained neural network module to perform a doze-off stage recognition operation according to the characteristic values, so as to obtain a doze-off stage recognition result.

A recognition method of doze-off stage is adapted to a wearable device. The wearable device includes a processor and an electrocardiogram sensor. The method includes: training a neural network module by the processor; generating an electrocardiogram signal by the electrocardiogram sensor; performing a heart rate variability analysis operation and a R-wave amplitude analysis operation by the processor to analyze a heart beat interval variation of the electrocardiogram signal, so as to generate a plurality of characteristic values; and utilizing the trained neural network module by the processor to perform a doze-off stage recognition operation according to the characteristic values, so as to obtain a doze-off stage recognition result.

Based on the above, the wearable device capable of recognizing doze-off stage and the method thereof according to the disclosure can sense a plurality of characteristic values by the electrocardiogram sensor. In this way, the processor can utilize the trained neural network module to perform the doze-off stage recognition operation according to the characteristic values. As a result, the wearable device of the disclosure can effectively obtain a recognition result of the doze-off stage and provide characteristics of convenience.

To make the above features and advantages of the disclosure more comprehensible, several embodiments accompanied with drawings are described in detail as follows.

### BRIEF DESCRIPTION OF THE DRAWINGS

The accompanying drawings are included to provide a further understanding of the disclosure, and are incorporated in and constitute a part of this specification. The drawings illustrate embodiments of the disclosure and, together with the description, serve to explain the principles of the disclosure.
FIG. 1 illustrates a block diagram of a wearable device in an embodiment of the disclosure.
FIG. 2 illustrates a waveform diagram of an electrocardiogram signal in an embodiment of the disclosure.
FIG. 3 illustrates a block diagram for analyzing the electrocardiogram signal in an embodiment of the disclosure.
FIG. 4 illustrates a waveform diagram of another plurality of characteristic values in an embodiment of the disclosure.
FIG. 5 illustrates a waveform diagram of a plurality of characteristic values in an embodiment of the disclosure.
FIG. 6 illustrates a flowchart of a recognition method of doze-off stage in an embodiment of the disclosure.

### DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

In order to make content of the disclosure more comprehensible, embodiments are provided below to describe the disclosure in detail, however, the disclosure is not limited to the provided embodiments, and the provided embodiments can be suitably combined. Moreover, elements/components/steps with same reference numerals represent same or similar parts in the drawings and embodiments.

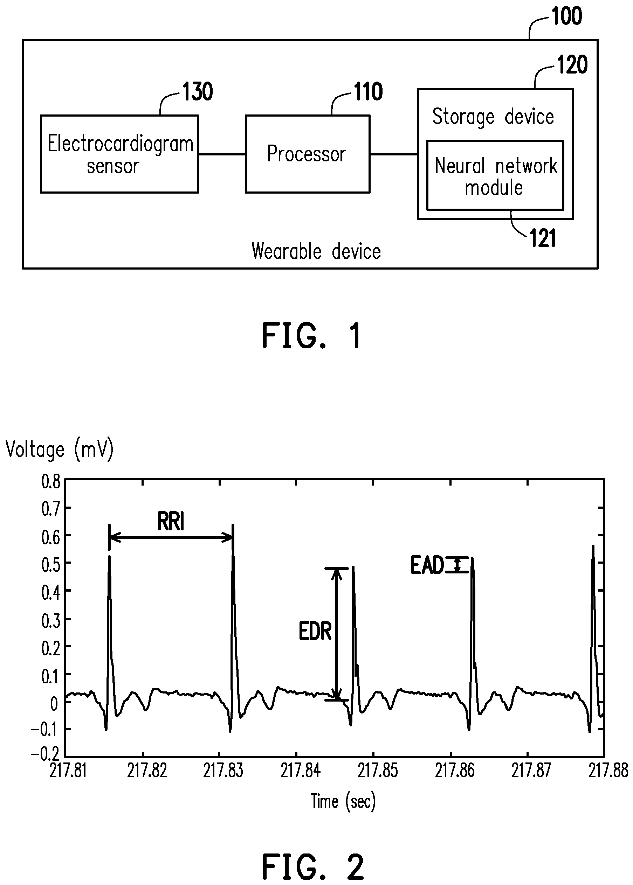

FIG. 1 illustrates a block diagram of a wearable device in an embodiment of the disclosure. With reference to FIG. 1, a wearable device 100 includes a processor 110, a storage device 120 and an electrocardiogram sensor 130. The processor 110 is coupled to the storage device 120 and the electrocardiogram sensor 130. In the present embodiment, the wearable device 100 may be, for example, smart clothes, smart wristbands or other similar devices, and the wearable device 100 is configured to recognize the doze-off stage of the wearer. In an embodiment, the doze-off stage may refer to a short afternoon nap. The wearable device 100 can integrate each of the sensors into one wearable item instead of complicated accessories. In other words, when the wearer is dozing off, the doze-off stage of the wearer may be sensed by the wearable device 100 worn by the wearer. In the present embodiment, a sleep recognition for the doze-off stage may be conducted for the wearer in a lying or sitting posture, for example, so as to monitor a sleep state of the wearer and record a sleep situation for the doze-off stage. Further, in the present embodiment, the doze-off stage recognizable by the wearable device 100 can include a wakefulness stage (Stage W) and a first non-rapid eye movement stage (Stage N1, a.k.a. falling-asleep stage). It should be noted that, a usage scenario of the wearable device 100 of the disclosure refers to a sleep situation of the wearer in a short period of time.

Incidentally, the sleep stage established by polysomnography standard includes the wakefulness stage, the first non-rapid eye movement stage, a second non-rapid eye movement stage (Stage N2, a.k.a. slightly-deeper sleep stage), a third non-rapid eye movement stage (Stage N3, a.k.a. deep sleep stage) and a rapid eye movement (Stage R). Nonetheless, the wearable device 100 of the disclosure is adapted to recognize the doze-off stage when the wearer is dozing off. As such, the doze-off stage to be recognized by the wearable device 100 of the disclosure with the trained neural network module only needs to include the wakefulness stage and the first non-rapid eye movement stage.

In the present embodiment, the electrocardiogram sensor 130 is configured to sense an electrocardiogram signal ECG and provide the electrocardiogram signal to the processor 110. The processor 110 analyzes the electrocardiogram signal to generate a plurality of characteristic values. In other words, when the wearer is asleep, because the wearer is less likely to turn over or move his/her body, the wearable device 100 of the present embodiment can simply recognize the doze-off stage of the wearer by sensing only the electrocardiogram information of the wearer.

In the present embodiment, the processor 110 is, for example, a central processing unit (CPU), a system on chi (SOC) or other programmable devices for general purpose or special purpose, such as a microprocessor and a digital signal processor (DSP), a programmable controller, an application specific integrated circuit (ASIC), a programmable logic device (PLD) or other similar devices or a combination of above-mentioned devices.

In the present embodiment, the storage device 120 is, for example, a dynamic random access memory (DRAM), a flash memory or a non-volatile random access memory (NVRAM). In the present embodiment, the storage device 120 is configured to store data and program modules described in each embodiment of the disclosure, which can be read and executed by the processor 110 so the wearable device 100 can realize the recognition method of sleep stage described in each embodiment of the disclosure.

In the present embodiment, the storage device 120 further includes a neural network module 121. The processor 110 can sense a plurality of characteristic values from different wearers in advance by the electrocardiogram sensor 130, and use the characteristic values from the different wearers as sample data. In the present embodiment, the processor 110 can create a prediction model according to determination conditions, algorithms and parameters from the doze-off stage, and use a plurality of the sample data for training or correcting the prediction model. Accordingly, when the doze-off stage recognition operation is performed by the wearable device 100 for the wearer, the processor 110 can utilize the trained neural network module 121 to obtain a recognition result according to the characteristic values sensed by the electrocardiogram sensor 130. Nevertheless, enough teaching, suggestion, and implementation illustration regarding algorithms and calculation modes for the trained neural network module 121 of the present embodiment may be obtained with reference to common knowledge in the related art, which is not repeated hereinafter.

FIG. 2 illustrates a waveform diagram of an electrocardiogram signal in an embodiment of the disclosure. With reference to FIG. 1 and FIG. 2, the electrocardiogram signal sensed by the electrocardiogram sensor 130 is as shown in FIG. 2. In the present embodiment, the processor 110 can perform a heart rate variability (HRV) analysis operation to analyze a heart beat interval variation (R-R intervals) RRI of the electrocardiogram signal, so as to obtain a plurality R-wave signals in the electrocardiogram signal. The processor 110 can perform the heart rate variability analysis operation to analyze variation of the R-wave signals. Further, the processor 110 can also perform a R-wave amplitude analysis operation and an adjacent R-waves difference (Difference of Amplitude between R and next R, EAD) analysis operation to analyze the R-wave signals. For instance, in the present embodiment, a distance between two R-waves may be used as the heart beat interval variation RRI. The R-wave amplitude analysis operation is, for example, to analyze a R-wave amplitude EDR in the electrocardiogram in order to obtain an ECG-derived respiratory signal, wherein peak and trough of the R-wave may be used as the R-wave amplitude EDR. A difference between the peaks of two adjacent R-waves may be used as the adjacent R-waves difference.

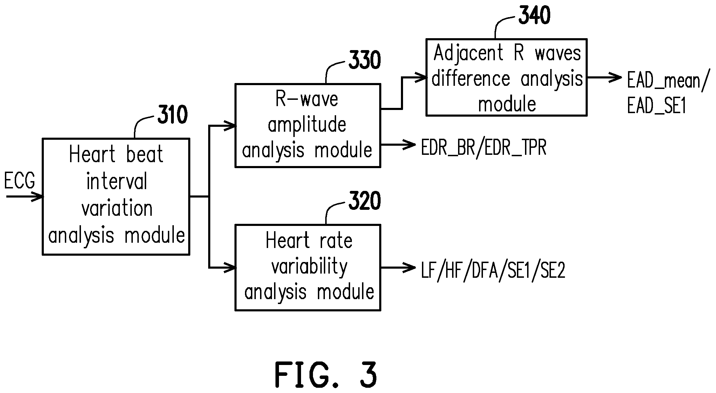

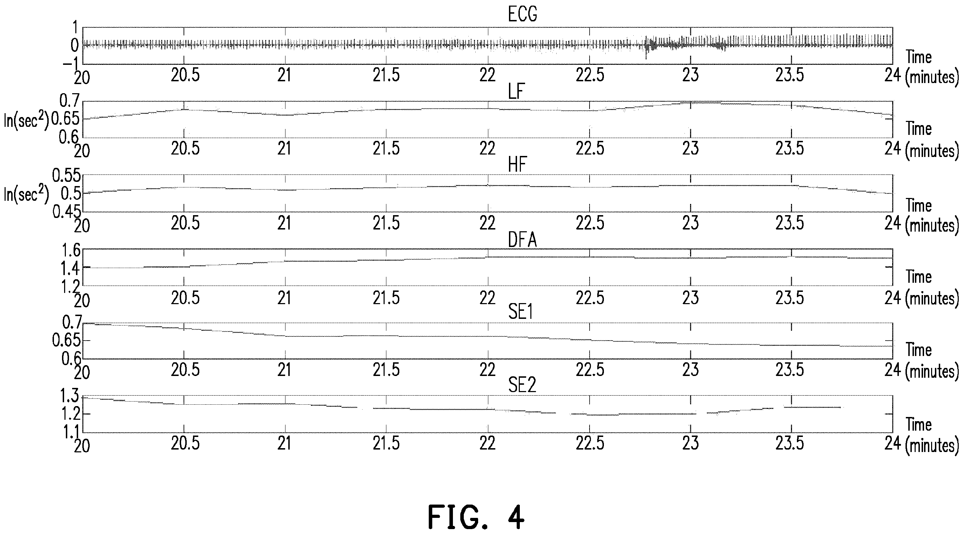

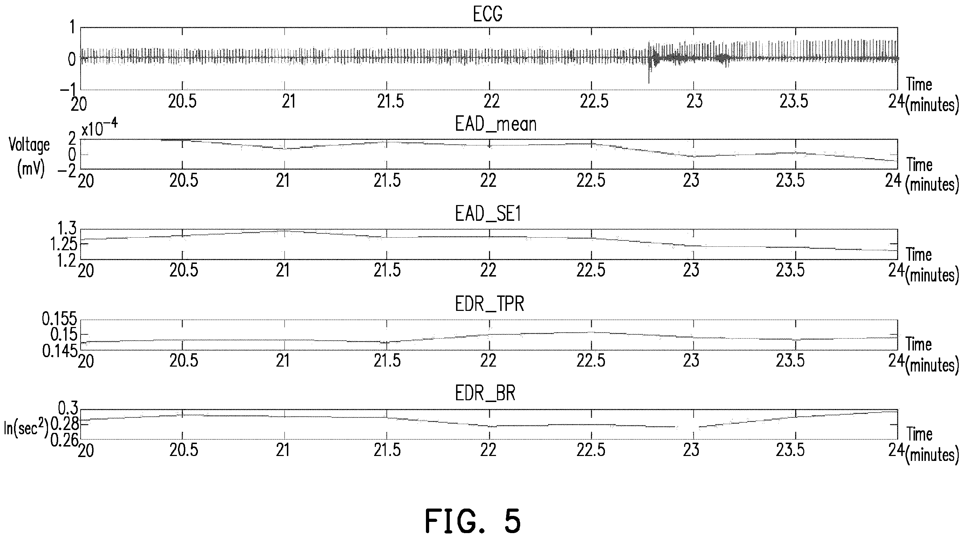

FIG. 3 illustrates a block diagram for analyzing the electrocardiogram signal in an embodiment of the disclosure. FIG. 4 illustrates a waveform diagram of a plurality of characteristic values in an embodiment of the disclosure. FIG. 5 illustrates a waveform diagram of another plurality of characteristic values in an embodiment of the disclosure. It should be noted that, each of the following waveform diagrams shows, for example, a sleep recognition operation performed per 30 seconds within a time length of 5 minutes. With reference to FIG. 1 to FIG. 5, the storage device 120 can store, for example, a heart beat interval variation analysis module 310, a heart rate variability analysis module 320, a R-wave amplitude analysis module 330 and an adjacent R-waves difference analysis module 340. In the present embodiment, the processor 110 receives the electrocardiogram signal ECG of the wearer provided by the electrocardiogram sensor, and analyzes the electrocardiogram signal ECG by the heart beat interval variation analysis module 310, so as to obtain a plurality of R-wave signal as shown in FIG. 2.

In the present embodiment, the heart rate variability analysis module 320 analyzes the R-wave signals, so as to obtain a low frequency signal LF, a high frequency signal HF, a detrended fluctuation analysis signal DFA, a first sample entropy signal SE1 and a second sample entropy signal SE2 as shown in FIG. 4. In the present embodiment, the low frequency signal LF is, for example, a signal with strength ranged from 0.04Hz to 0.15Hz among the R-wave signals. The high frequency signal HF is, for example, a signal with strength ranged from 0.15Hz to 0.4Hz among the R-wave signals. The detrended fluctuation analysis signal DFA is, for example, a signal underwent a detrended fluctuation analysis (DFA) among the R-wave signals. The first sample entropy signal SE1 is, for example, a signal underwent a sample entropy operation with the number of samples being 1 among the R-wave signals. The second sample entropy signal SE2 is, for example, a signal underwent a sample entropy operation with the number of samples being 2 among the R-wave signals.

In the present embodiment, the R-wave amplitude analysis module 330 analyzes the R-wave signals, so as to obtain a result including a turning point ratio value EDR_TPR and a signal strength value EDR_BR as shown in FIG. 5. Further, in the present embodiment, the adjacent R-waves difference analysis module 340 analyzes the R-wave signals, so as to obtain a mean value EAD_mean and a sample entropy value EAD_SE1 as shown in FIG. 5. In other words, the characteristic values of the present embodiment are obtained from the low frequency signal LF, the high frequency signal HF, the detrended fluctuation analysis signal DFA, the first sample entropy signal SE1 and the second sample entropy signal SE2, and include the turning point ratio value EDR_TPR, the signal strength value EDR_BR, mean value EAD_mean and a sample entropy value EAD_SE1.

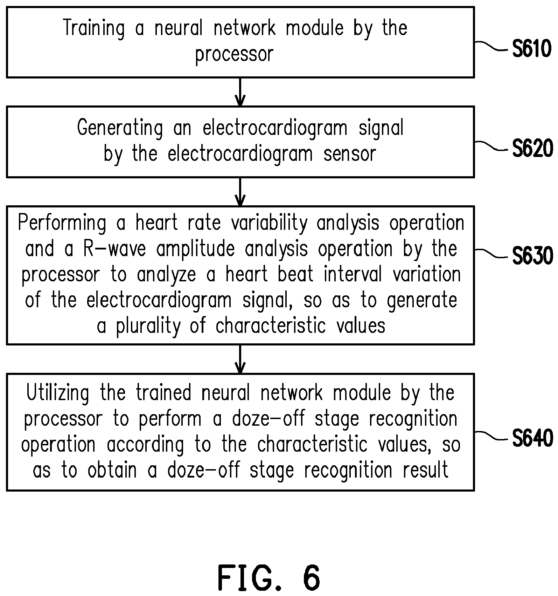

FIG. 6 illustrates a flowchart of a recognition method of doze-off stage in an embodiment of the disclosure. With reference to FIG. 1 and FIG. 6, the recognition method of the present embodiment is at least adapted to the wearable device 100 of FIG. 1. In step S610, the processor 110 trains the neural network module 121. In step S620, the electrocardiogram sensor 130 generates an electrocardiogram signal. In step S630, the processor 110 performs a heart rate variability analysis operation and a R wave amplitude analysis operation to analyze a heart beat interval variation of the electrocardiogram signal, so as to generate a plurality of characteristic values. In step S640, the processor 110 utilizes the trained neural network module 121 to perform a doze-off stage recognition operation according to the characteristic values, so as to obtain a doze-off stage recognition result.

In this way, the recognition method of the present embodiment can recognize the doze-off stage of the wearer according to said 9 characteristic values sensed by the electrocardiogram sensor 130. In the present embodiment, the doze-off stage recognition result is one of the wakefulness stage and the first non-rapid eye movement stage. Also, the wakefulness stage and the first non-rapid eye movement stage are established by a polysomnography standard.

In addition, sufficient teaching, suggestion, and implementation regarding detailed features of each module and each sensor in the wearable device 100 of the present embodiment the disclosure may be obtained from the foregoing embodiments of FIG. 1 to FIG. 5, and thus related descriptions thereof are not repeated hereinafter.

In summary, the wearable device capable of recognizing doze-off stage and the recognition method of doze-off stage according to the disclosure can provide an accurate sleep recognition function. The wearable device includes the electrocardiogram sensor so the wearable device can sense the electrocardiogram information of the wearer. The wearable device of the disclosure can use the trained neural network module to perform the doze-off stage recognition operation according to the characteristic values provided by the electrocardiogram sensor. Moreover, the wearable device of the disclosure can integrate each of the sensors into one wearable item instead of complicated accessories. As a result, the wearable device of the disclosure is suitable for the wearer to conveniently and effectively monitor the sleep state in a home environment during the doze-off stage so as to obtain the recognition result of the doze-off stage of the wearer.

## Claims

1. A wearable device (100) capable of recognizing doze-off stage, comprising:
a processor (110), configured to train a neural network module; and
an electrocardiogram sensor (130), coupled to the processor (110), and configured to generate an electrocardiogram signal (ECG), wherein the processor (110) performs a heart rate variability analysis operation and a R-wave amplitude analysis operation to analyze a heart beat interval variation of the electrocardiogram signal (ECG), so as to generate a plurality of characteristic values,
wherein the processor (110) utilizes the trained neural network module (121) to perform a doze-off stage recognition operation according to the characteristic values, so as to obtain a doze-off stage recognition result.

2. The wearable device (100) according to claim 1, wherein the processor (110) performs the heart rate variability analysis operation to analyze the heart beat interval variation of the electrocardiogram signal (ECG), so as to obtain a low frequency signal (LF), a high frequency signal (HF), a detrended fluctuation analysis signal (DFA), a first sample entropy signal (SE1) and a second sample entropy signal (SE2),
wherein the characteristic values are obtained from the low frequency signal (LF), the high frequency signal (HF), the detrended fluctuation analysis signal (DFA), the first sample entropy signal (SE1) and the second sample entropy signal (SE2).

3. The wearable device (100) according to claim 1 or 2, wherein the processor (110) performs the R-wave amplitude analysis operation to analyze the heart beat interval variation of the electrocardiogram signal (ECG), so as to obtain a turning point ratio value (EDR_TPR) and a signal strength value (EDR_BR),
wherein the characteristic values comprise the turning point ratio value (EDR_TPR) and the signal strength value (EDR_BR).

4. The wearable device (100) according to claim 3, wherein the processor (110) performs an adjacent R-waves difference analysis operation to analyze the heart beat interval variation of the electrocardiogram signal (ECG), so as to obtain a mean value (EAD_mean) and a sample entropy value (EAD_SE1),
wherein the characteristic values comprise the mean value (EAD_mean) and the sample entropy value (EAD_SE1).

5. The wearable device (100) according to any one of claims 1 to 4, wherein the doze-off stage recognition result is a wakefulness stage or a first non-rapid eye movement stage, and wakefulness stage and the first non-rapid eye movement stage are established by a polysomnography standard.

6. The wearable device (100) according to any one of claims 1 to 5, wherein the processor (110) pre-trains the neural network module (121) according to a plurality of sample data, and each of the sample data comprises another plurality of characteristic values.

7. A recognition method of doze-off stage, adapted to a wearable device (100), the wearable device (100) comprising a processor (110) and an electrocardiogram sensor (130), the method comprising:
training a neural network module by the processor (110);
generating an electrocardiogram signal (ECG) by the electrocardiogram sensor (130);
performing a heart rate variability analysis operation and a R-wave amplitude analysis operation by the processor (110) to analyze a heart beat interval variation of the electrocardiogram signal (ECG), so as to generate a plurality of characteristic values; and
utilizing the trained neural network module (121) by the processor (110) to perform a doze-off stage recognition operation according to the characteristic values, so as to obtain a doze-off stage recognition result.

8. The recognition method of doze-off stage according to claim 7, wherein the step of performing the heart rate variability analysis operation and the R-wave amplitude analysis operation by the processor (110) to analyze the heart beat interval variation of the electrocardiogram signal (ECG), so as to generate the characteristic values comprises:
performing the heart rate variability analysis operation by the processor (110) to analyze the heart beat interval variation of the electrocardiogram signal (ECG), so as to obtain a low frequency signal (LF), a high frequency signal (HF), a detrended fluctuation analysis signal (DFA), a first sample entropy signal (SE1) and a second sample entropy signal (SE2),
wherein the characteristic values are obtained from the low frequency signal (LF), the high frequency signal (HF), the detrended fluctuation analysis signal (DFA), the first sample entropy signal (SE1) and the second sample entropy signal (SE2).

9. The recognition method of doze-off stage according to claim 7 or 8, wherein the step of performing the heart rate variability analysis operation and the R-wave amplitude analysis operation by the processor (110) to analyze the heart beat interval variation of the electrocardiogram signal (ECG), so as to generate the characteristic values comprises:
performing the R-wave amplitude analysis operation by the processor (110) to analyze the heart beat interval variation of the electrocardiogram signal (ECG), so as to obtain a turning point ratio value (EDR_TPR) and a signal strength value (EDR_BR),
wherein the characteristic values comprise the turning point ratio value (EDR_TPR) and the signal strength value (EDR_BR).

10. The recognition method of doze-off stage according to claim 9, wherein the step of performing the heart rate variability analysis operation and the R-wave amplitude analysis operation by the processor (110) to analyze the heart beat interval variation of the electrocardiogram signal (ECG), so as to generate the characteristic values further comprises:
performing an adjacent R-waves difference analysis operation by the processor (110) to analyze the heart beat interval variation of the electrocardiogram signal (ECG), so as to obtain a mean value (EAD_mean) and a sample entropy value (EAD_SE1),
wherein the characteristic values comprise the mean value (EAD_mean) and the sample entropy value (EAD_SE1).

11. The recognition method of doze-off stage according to any one of claims 7 to 10, wherein the doze-off stage recognition result is a wakefulness stage or a first non-rapid eye movement stage, and wakefulness stage and the first non-rapid eye movement stage are established by a polysomnography standard.

12. The recognition method of doze-off stage according to any one of claims 7 to 11, wherein the processor (110) pre-trains the neural network module according to a plurality of sample data, and each of the sample data comprises another plurality of characteristic values.
